# FASCICULE DE BREVET EUROPEEN

(11) **EP 0 719 861 B1**
(45) Date de publication et mention de la délivrance du brevet: **13.02.2008**
(21) Numéro de dépôt: 95116846.7
(22) Date de dépôt: 11.07.1991
(51) Int. Cl.: C12N 15/31, C07K 2/00, C12Q 1/68, G01N 33/569

(54) **Séquences nucléics issues du génome de Salmonella Typhi, et leurs utilisations notamment pour le diagnostic in vitro de la présence de bactéries du genre salmonella dans les produits alimentaires**
Salmonella Genom Nukleinsäure, ihre Verwendung, besonders bei der in vitro Diagnose der Anwesenheit von Bakterien der Gattung Salmonella in Lebensmitteln
Nucleic sequences from the genome of Salmonella Typhi, and uses thereof particularly for the in vitro diagnosis of the presence of salmonella bacteria in foodstuffs

(30) Priorité: 11.07.1990 FR 9008852
(43) Date de publication de la demande: 03.07.1996
(62) Demande divisionnaire de: 91913033.6
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Popoff, Michel Yvan, F-78370 Plaisir (FR); Dion, Michel Pierre, F-75013 Paris (FR)
(74) Mandataire: Gutmann, Ernest

(56) Documents cités:
- EP-A- 0 114 668
- EP-A- 0 355 989
- PROCEEDINGS OF THE NATIONAL ACADEMY OF SCIENCES OF USA, vol. 84, Décembre 1987 WASHINGTON US, pages 9059-9063, FRIEND, S.H. ET AL.; 'Deletions of a DNA sequence in retinoblastomas and mesenchymal tumors : organisation of the sequence and its encoded protein.'

## Description

La présente invention a pour objet des séquences nucléiques et leurs utilisations notamment pour le diagnostic in vitro de la présence de bactéries du genre Salmonella dans un échantillon biologique susceptible de les contenir, et plus particulièrement dans les produits alimentaires.

Les produits alimentaires prennent actuellement une importance grandissante. Pour des motifs sociaux, les denrées transformées, prétes à la consommation, ne cessent de susciter une demande accrue de la part des acheteurs. Il en résulte que les sources de production et la présentation de ces produits se sont considérablement modifiées durant les quinze dernières années (fabrication de masse dans des usines spécialisées ; commercialisation en unités conditionnées, généralement sous pellicule plastique).

Pour des raisons de santé publique, la technologie des fabrications et les produits eux-mêmes sont soumis à une surveillance hygiénique de plus en plus stricte. Dans le cadre des contrôles bactériologiques, les bactéries responsables de toxi-infections alimentaires sont recherchées systématiquement, et parmi celles-ci, ce sont les Salmonella qui sont concernées en priorité. Les normes de salubrité sont d'ailleurs très claires pour ce genre bactérien : absence de Salmonella dans 25 grammes de produit.

Du point de vue de la taxonomie, le genre Salmonella appartient à la famille des Enterobacteriaceae. Ce genre comprend une seule et unique espèce, S.enterica, qui peut être subdivisée en sept sous-espèces. A l'intérieur de chacune de ces sous-espèces, on peut individualiser un grand nombre de sérotypes à l'aide de sérums dirigés contre les antigènes O polysaccharidiques et les antigènes H flagellaires. En 1989, on connaissait 2267 sérotypes de Salmonella.

Les Salmonella sont des bactéries entéro-in-vasives qui sont pathogènes pour l'homme et les animaux. Leur pouvoir pathogène est lié à leur capacité d'envahir l'épithélium intestinal. Cette étape peut se limiter à la muqueuse dans le cas d'une toxi-infection par exemple, ou être suivie d'une dissémination systémique (cas de la fièvre thyphoïde). La contamination de l'hôte par Salmonella a lieu par voie buccale dans la très grande majorité des cas. Ceci explique pourquoi les Salmonella sont recherchées systématiquement dans le cadre de contrôles bactériologiques d'échantillons biologiques.

La méthode de référence de recherche systématique de Salmonella, recommandée par l'Association Française de Normalisation (AFNOR), comprend : la mise en culture du produit à analyser dans deux milieux d'enrichissement différents, incubés à deux températures différentes et isolés sur deux milieux sélectifs différents (cette étape est souvent précédée d'une étape de "revivification" dans un bouillon nutritif) ; repiquage de 6 colonies au minimum sur milieux d'identification rapide ; enfin typage sérologique de la souche. Si le protocole AFNOR doit être scrupuleusement suivi lors d'une expertise officielle, on conçoit qu'il soit difficilement applicable lors de contrôles systématiques en raison du temps nécessaire pour effectuer l'examen (au minimum une semaine) et de son prix de revient.

Des nouvelles méthodes de recherche de Salmonella reposent soit sur des tests enzymatiques, soit sur l'utilisation de sondes nucléiques. Il faut souligner que dans les deux cas, une étape de culture en milieu riche et une subculture dans un milieu d'enrichissement sont recommandées, voire indispensables.

Les tests immuno-enzymatiques qui utilisent des anticorps monoclonaux, sont aisés à mettre en oeuvre, donnent des résultats en quatre à six heures et sont d'un prix de revient abordable. Leur gros inconvénient réside dans le fait qu'ils donnent souvent de fausses réactions positives et parfois de fausses réactions négatives. Les conséquences de ces fausses réactions sont importantes dans les deux cas : s'il s'agit d'une fausse réaction positive, il y aura saisie du produit et mise en route par le laboratoire d'analyse d'un processus pour isoler une Salmonella qui n'existe pas; s'il s'agit d'une fausse réaction négative, le produit sera commercialisé avec les risques que cela comporte pour la santé publique.

Une étude récente sur 250 souches de Salmonella et 75 souches bactériennes n'appartenant pas au genre Salmonella, a abouti à 17 fausses réactions positives et 2 fausses réactions négatives (D'AOUST, J.Y. (1987): "Efficacité de la trousse immunoenzymatique BIOENZABEAD pour le dépistage de Salmonella spp. dans les aliments", Microorganismes et Aliments : Technique Rapide, Contrôle industriel. Colloque de la Société Française de Microbiologie, Paris). Cet essai ayant été effectué sur des cultures pures, on peut penser que le nombre de fausses réactions auraient été plus important avec des produits poly-microbiens (compétition microbienne ; risque de réactions antigéniques croisées).

Les sondes nucléiques sont constituées par un fragment d'ADN génomique (FITTS, R. et al (1983), "DNA-DNA Hybridization Assay For Detection Of Salmonella spp". In Foods, Applied and Environmental Microbiology, 46, 1146-1151). Elles sont réputées spécifiques. Il existe plusieurs trousses (ou kits) commercialisées. En fait, il apparaît que ces sondes présentent deux inconvénients majeurs : leur manque de spécificité selon les résultats communiqués par des utilisateurs (réaction croisée avec les Citrobacter, par exemple) et leur manque de sensibilité (seuil limite de détection : 10⁵ Salmonella ; ou selon certains auteurs, 2,5 µg d'ADN soit environ 10⁷ bactéries).

La présente invention a précisément pour objet des séquences nucléiques utilisables en tant que sondes nucléiques pour la détection de Salmonella, ces sondes étant parfaitement spécifiques du genre Salmonella (pas de réaction croisée avec d'autres bactéries, notamment avec les bactéries du genre Citrobacter).

L'invention a également pour objet l'utilisation de ces sondes nucléiques dans des méthodes de détection ou de dosage in vitro de Salmonella présentant les avantages suivants :
- forte sensibilité,
- réponse rapide ; le résultats de l'analyse peut être rendu dans les 48 heures, voire 24 heures,
- mise en oeuvre aisée, notamment sans utilisation d'un produit radioactif,
- prix de revient raisonnable.

Salmonella sous-espèce enterica sérotype Typhi (bactérie désignée par Typhi ci-après) est l'agent de la fièvre typhoïde humaine. Cette bactérie est strictement pathogène pour l'homme. A la suite d'une contamination par voie buccale, Typhi va franchir la barrière intestinale pour atteindre les ganglions mésentériques grâce à un système génétique lui permettant d'adhérer et de pénétrer dans les cellules épithéliales de la muqueuse intestinale. Faute de modèle expérimental, cette première étape de l'infection est étudiée in vitro sur cellules HeLa en culture, car Typhi est capable d'ahérer et de pénétrer dans ces cellules.

L'invention a précisément pour objet un acide nucléique isolé susceptible d'être utilisé comme sonde et/ou amorce pour la détection *in vitro* de bactéries du genre Salmonella, caractérisé en ce qu'il est susceptible de s'hybrider à 65°C en 6x SSC en milieu Denhardt, avec un fragment HindIII de 7,9 kb de la souche *Salmonella Typhi* Ty2 comprenant l'enchaînement nucléotidique (I) suivant:

1
5' >CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

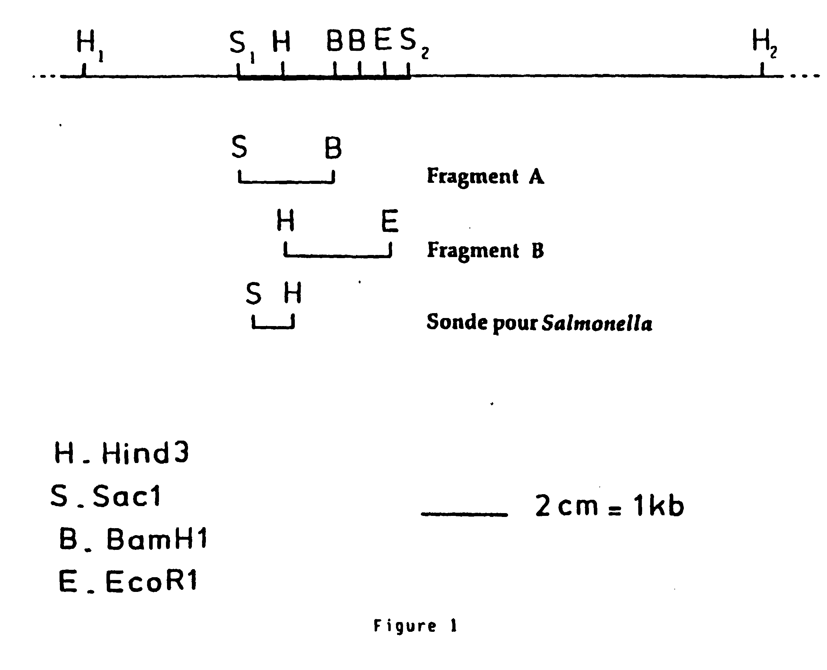

L'invention a plus précisément pour objet toute séquence nucléique telle que définie ci-dessus, et comprenant tout ou partie de la séquence de 7,9 kb délimitée par les deux sites HindIII, désignés par H₁ et H₂ sur la carte de restriction de ladite séquence représentée sur la figure 1. Les séquences avec lesquelles les acides nucléiques de l' invention hybrident sont issues du génome de la souche Ty 2 de Typhi déposée à la Collection de l'Institut Pasteur sous le n° CIP 55-35.

Ainsi, la demande décrit toute séquence nucléique issue du génome de la souche Ty 2 sus-mentionnée, cette séquence comprenant toute ou partie de la séquence de 2 kb délimitée par les deux sites SacI, désignés par S₁ et S₂ sur la carte de restriction représentée sur la figure1.

D'une manière plus générale, la demande divulgué toute séquence nucléique comportant toute ou partie de l'information génétique nécessaire à l'activité d'infection in vitro des cellules HeLa en culture par les bactéries du genre Salmonella, et susceptible de s'hybrider avec toute ou partie de l'une au moins des séquences nucléiques définies ci-dessus, dans des conditions stringentes. Ces conditions stringentes sont les suivantes : 65°C durant 18 heures en 6 × SSC (1xSSC est constitué de 0,15M NaCl et 0,015M citrate trisodique à pH 7) en milieu de Denhardt (0,1% Ficoll ; 0,1 % de polyvynil-pyrrolidone ; 0,1 % de sérum albumine de boeuf).

L'invention concerne également toute séquence nucléique susceptible de s'hybrider avec l'une des séquences définies ci-dessus, dans les conditions d'hybridation définies ci-dessus, et étant utilisable en tant que sonde pour la mise en oeuvre d'un procédé de détection in vitro de bactéries du genre Salmonella, et plus particulièrement des Salmonella pathogènes telles que Typhi, susceptibles d'adhérer et de pénétrer dans les cellules épithéliales de la muqueuse intestinale dans l'organisme.

Avantageusement les sondes nucléiques de Δ l'invention sont constituées d'une succession d'environ 200 à 500 nucléotides.

A ce titre, la demande décrit une sonde nucléique caractérisée par l'enchaînement nucléotidique (I) suivant :

1
5'> CAATTACGCCCCGATTATTATATCTCCGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

L' invention concerne également toute séquence nucléique susceptible de s'hybrider avec l'une des séquences nucléiques définies ci-dessus dans les conditions d'hybridation définies ci-dessus, et étant utilisable en tant qu'amorce nucléique pour l'amplification génique d'une des séquences nucléiques décrites dans la demande.

Avantageusement les amorces nucléiques de l'invention sont constituées d'une succession d'environ 10 à 30 nucléotides.

Les techniques d'amplification génique sont d'un appoint considérable pour la mise au point de méthodes de diagnostic in vitro particulièrement sensibles. Parmi ces techniques d'amplification génique, on peut citer la technique PCR (Polymerase Chain Reaction) telle que décrite dans les demandes de brevet européen n° 86/302.298.4 du 27/03/1986 et n° 87/300.203.4 du 09/01/1987, ou encore la technique dite "Qβreplicase" décrite dans Biotechnology, vol. 6, page 1197 (octobre 1988) et celle procédant à l'aide d'une ARN polymérase (T7RNA polymérase) décrite dans la demande de brevet international n° WO89/01050. Ces techniques permettent d'améliorer la sensibilité de détection des acides nucléiques des virus ou des bactéries, et nécessitent l'utilisation d'amorces de synthèse spécifiques.

Font également partie de l'invention, les acides nucléiques variants par rapport à la séquence nucléique (I) sus-définie et qui comportent certaines mutations localisées dans la mesure où ces acides nucléiques variants s'hybrident avec la séquence (I) définie dans les conditions d'hybridation définies ci-dessus dans la description. La demande décrit également tout peptide ou polypeptide correspondant selon le code génétique universel à une séquence nucléique de l'invention.

A ce titre, la demande divulgue plus particulièrement tout polypeptide constitué par toute ou partie de la séquence en acides aminés (II) suivante, cette séquence correspondant selon le code génétique universel à la séquence nucléique (I) décrite ci-dessus :

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

Il va de soi que toute séquence peptidique issue de la modification, par substitution et/ou par addition et/ou suppression d'un ou plusieurs acides aminés d'un polypeptide de la demande, et plus particulièrement de la séquence peptidique (II) décrite ci-dessus) ou d'une sous-séquence peptidique issue de cette dernière sont divulgués dans le cadre de la présente demande dès lors que cette modification n'altère pas les propriétés antigéniques dudit polypeptide. La demande décrit également les anticorps pplyclonaux ou monoclonaux, susceptibles de reconnaître spécifiquement les polypeptides de décrits ci-dessus, et de former des complexes immunologiques avec ces derniers.

Ces anticorps polyclonaux sont obtenus par immunisation d'un animal avec les polypeptides, suivie de la récupération des anticorps formés.

Ces anticorps monoclonaux sont produits par tout hybridome susceptible d'être formé, par des méthodes classiques, à partir des cellules spléniques d'un animal, notamment de souris ou de rat, immunisés contre l'un des polypeptides purifiés décrits dans la demande l'invention, d'une part et des cellules d'une lignée de cellules d'un myélome approprié d'autre part, et d'être sélectionné, par sa capacité à produire des anticorps monoclonaux reconnaissant le polypeptide initialement mis en oeuvre pour l'immunisation des animaux.

L'invention concerne également un procédé de détection (ou méthode de diagnostic) in vitro de la présence éventuelle de bactéries du genre Salmonella dans un échantillon biologique susceptible de les contenir, caractérisé en ce qu'il comprend :
- le cas échéant, la mise en culture de l'échantillon,
- le cas échéant, l'amplification du nombre de copies de la séquence nucléique à détecter à l'aide d'un couple d'amorces nucléiques telles que définies ci-dessus,
- la mise en contact de l'échantillon avec une sonde nucléique telle que définie ci-dessus, dans les conditions d'hybridation sus-mentionnées,
- la détection éventuelle de complexes d'hybridation formés entre la sonde sus-mentionnée et la séquence nucléique à détecter.

L'étape de mise en culture de l'échantillon biologique est avantageusement réalisée de la manière suivante : 25 g de l'échantillon biologique sont mis en culture dans 200 ml de bouillon nutritif dans un erlen-meyer de 1 litre durant 18 h à 37°C avec agitation.

L'étape d'amplification de la séquence nucléique à détecter, dans le procédé sus-mentionné, comprend avantageusement les étapes suivantes :
- une étape d'extraction de l'acide nucléique à détecter appartenant au génome des bactéries du genre Salmonella éventuellement présentes dans l'échantillon biologique, et,le cas échéant, une étape de traitement à l'aide d'une transcriptase inverse dudit acide nucléique si ce dernier est sous forme d'ARN,
- un cycle comprenant les étapes suivantes :
   - étape de dénaturation de l'acide nucléique double brin à détecter , ce qui conduit à la formation d'un acide nucléique simple brin ; cette étape est avantageusement réalisée à 94°C pendant 120 secondes,
   - étape de réassociation par hybridation de chacun des brins d'acide nucléique, obtenus lors de l'étape de dénaturation précédente, avec au moins une amorce telle que définie ci-dessus, par mise en contact des brins sus-mentionnés avec au moins un couple d'amorces sus-mentionnées ; cette étape est avantageusement réalisée à 68°C pendant 180 secondes,
   - étape d'élongation par formation à partir des amorces des ADN complémentaires aux brins sur lesquels elles sont hybridées en présence d'une ADN polymérase et de quantités appropriées des quatre nucléosides triphosphate différents (dATP, dCTP, dGTP, dTTP), ce qui conduit à la formation d'un plus grand nombre d'acides nucléiques double brin à détecter qu'à l'étape de dénaturation précédente : cette étape est avantageusement réalisée à 72°C pendant 90 secondes,
   ce cycle étant répété un nombre de fois déterminé (de préférence entre 20 et 30 fois) pour obtenir ladite séquence nucléique à détecter éventuellement présente dans l'échantillon biologique dans une proportion suffisante pour permettre sa détection.

D'autres méthodes d'amplification et/ou de détection des séquences nucléiques caractéristiques des Salmonella, utilisant les sondes, et le cas échéant, les amorces nucléiques décrites ci-dessus, sont utilisables dans le cadre de la présente invention. A ce titre on peut citer la méthode décrite dans la demande de brevet internationale WO 85/06700, ou encore celle décrite dans la demande de brevet européen n° 0357336.

L'invention a également pour objet des kits pour la mise en oeuvre d'un procédé de détection in vitro de bactéries du genre Salmonella tel que décrit ci-dessus, ces kits comprenant :
- le cas échéant, un milieu approprié pour la mise en culture de l'échantillon biologique,
- au moins un couple d'amorces nucléiques décrites ci-dessus,
- le cas échéant, des réactifs appropriés à la mise en oeuvre du cycle d'amplification, notamment de l'ADN (ou ARN) polymérase, et des quantités appropriées des 4 nucléosides triphosphate différents,
- une (ou plusieurs) sonde(s) nucléique(s) telle(s) que décrite(s) ci-dessus, pouvant être marquée, capable de s'hybrider avec la (ou les) séquence(s) nucléique(s) à détecter,
- des réactifs appropriés à la mise en oeuvre de la réaction d'hybridation entre la (ou les) sonde(s) et la (ou les) séquence(s) nucléique(s) à détecter sus-mentionnées.
- un tissu ou fluide biologique de référence dépourvu de séquences nucléiques susceptibles de s'hybrider avec la (ou les) sonde(s) sus-mentionnée(s).

La demande divulgue également un procédé de détection in vitro de la présence éventuelle de bactéries de genre Salmonella dans un échantillon biologique susceptible de les contenir, caractérisé en ce qu'il comprend :
- le cas échéant, la mise en culture de l'échantillon biologique de la manière indiquée ci-dessus,
- le cas échéant, l'amplification du nombre de copies de la séquence nucléique correspondant selon le code génétique universel au polypeptide à détecter (cette amplification étant réalisée suivant le cycle d'amplification décrit ci-dessus),
- la mise en contact de l'échantillon sus-mentionné avec des anticorps de l'invention susceptibles de former un complexe immunologique avec le (ou les) polypeptide(s) à détecter,
- la détection éventuelle des complexes immunologiques formés entre lesdits anticorps et la (ou les) séquence(s) peptidique(s) à détecter.

L'invention décrit également des kits pour la mise en oeuvre du procédé de détection décrit ci-dessus, qui comprennent :
- le cas échéant, un milieu approprié pour la mise en culture de l'échantillon biologique,
- le cas échéant, un couple d'amorces décrites ci-dessus et des réactifs appropriés à la mise en oeuvre du cycle d'amplification, notamment de l'ADN (ou ARN) polymérase et des quantités appropriées des 4 nucléosides triphosphate différents,
- des anticorps, polyclonaux ou monoclonaux, pouvant être marqués, notamment de manière radioactive ou enzymatique, avec la (ou les) séquence(s) peptidique(s) à détecter,
- les réactifs pour la constitution du milieu propice à la réalisation de la réaction immunologique entre les anticorps et la (ou les) séquence(s) peptidique(s) sus-mentionnés,
- les réactifs permettant la détection des complexes immunologiques formés lors de la susdite réaction immunologique. De tels réactifs peuvent également porter un marqueur ou être susceptibles d'être reconnus à leur tour par un réactif marqué,
- un tissu ou fluide biologique de référence dépourvu de polypeptides susceptibles d'être reconnus par les anticorps sus-mentionnés.

La demande enseigne également un procédé de préparation des séquences nucléiques décrites ci-dessus, ce procédé comprenant les étapes suivantes:
- incubation de l'ADN génomique, isolé à partir d'une bactérie Salmonella Typhi, par traitement par la soude à pH 12,5
- traitement de l'ADN ainsi extrait par une endonucléase appropriée,
- le clonage des acides nucléiques ainsi obtenus dans un vecteur approprié et la récupération de l'acide nucléique recherché à l'aide d'une sonde appropriée choisie parmi celles décrites ci-dessus.

Un procédé de préparation particulièrement avantageux des séquences nucléiques de l'invention comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée choisie parmi celles décrites ci-dessus.

Un autre procédé de préparation des séquences nucléotidiques de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée choisie parmi celles décrites ci-dessus.

L'invention a également pour objet tout acide nucléique recombinant contenant au moins une séquence nucléique de l'invention, insérée dans un acide nucléique hétérologue vis-à-vis de ladite séquence nucléique.

L'invention concerne plus particulièrement un acide nucléique recombinant tel que défini ci-dessus, dans lequel la séquence nucléique de l'invention est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

L'invention concerne tout vecteur recombinant, utilisé en particulier pour le clonage d'une séquence nucléique de l'invention, et/ou l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, ou les phages.

La demande décrit également un procédé de préparation d'un polypeptide de l'invention, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, l'invention concerne tout hôte cellulaire transformé par un vecteur recombinant tel que défini ci-dessus, et comprenant les éléments de régulation permettant l'expression de la séquence nucléique codant pour un polypeptide selon l'invention.

La demande décrit également plus particulièrement un procédé de préparation d'un polypeptide de l'invention comprenant les étapes suivantes :
- le cas échéant, l'amplification de la quantité de séquences de nucléotides codant pour ledit polypeptide à l'aide de deux amorces d'ADN choisies de manière à ce que l'une de ces amorces soit identique aux 10 à 30 premiers nucléotides de la séquence nucléotidique codant pour ledit polypeptide, tandis que l'autre amorce est complémentaire des 10 à 30 derniers nucléotides (ou s'hybride avec ces 10 à 30 derniers nucléotides) de ladite séquence nucléotidique, ou inversement de manière à ce ce que l'une de ces amorces soit identique aux 10 à 30 derniers nucléotides de ladite séquence, tandis que l'autre amorce est complémentaire des 10 à 30 premiers nucléotides (ou s'hybride avec les 10 à 30 premiers nucléotides) de ladite séquence nucléotidique, suivie de l'introduction desdites séquences de nucléotides ainsi amplifiées dans un vecteur approprié,
- la mise en culture, dans un milieu de culture approprié, d'un hôte cellulaire préalablement transformé par un vecteur approprié contenant un acide nucléique selon l'invention comprenant la séquence nucléotidique codant pour ledit polypeptide, et
- la récupération, à partir du susdit milieu de culture du polypeptide produit par ledit hôte cellulaire transformé.

Les peptides décrits dans la demande peuvent être préparés par les techniques classiques, dans le domaine de la synthèse des peptides. Cette synthèse peut être réalisée en solution homogène ou en phase solide.

Par exemple, on aura recours à la technique de synthèse en solution homogène décrite par HOUBENWEYL dans l'ouvrage intitulé "Methode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorydrique.

L'invention sera davantage illustrée à l'aide des exemples contenus dans la description détaillée qui suit, cette dernière ne révêtant aucun caractère limitatif.

### 1. Isolement du fragment BacI de 2 Kb

Le but de la présente invention est le clonage du système adhésion-invasion de Typhi.

La souche utilisée est la souche Ty 2 de Typhi déposée à la Collection de L'INSTITUT PASTEUR sous la référence CIP 55-35.

Partant d'une collection de 2000 mutants indépendants obtenus par insertion d'un transposon dérivé de Tn5 (MANOIL C. et BECKWITH J., 1985. TnPhoA:a transposon probe for protein export signals. Proc. Natl. Acad. Sci. USA, 82, 8129-8133) dans le génome de la souche Typhi Ty 2, il a été obtenu un mutant qui n'était plus invasif dans le modèle de cellules HeLa en culture. La technique d'infection des cellules HeLa en culture par Typhi est la suivante:les cellules HeLa sont cultivées en milieu RPMI 1640 contenant 10 % de sérum de veau foetal. Ces cellules sont infectées par Typhi à un ratio de 100 bactéries par cellule. Après une heure d'incubation à 37°C, les cellules sont lavées avec le milieu de culture, puis remises en culture en présence de gentamycine à 100 µg/ml. Les bactéries intracellulaires sont détectées après 24 h par une coloration de Giemsa.

Après digestion du génome de ce mutant par l'endonucléase de restriction SacI, des expériences d'hybridation sur feuille de nitrate de cellulose ont montré que le transposon était inséré dans un fragment SacI de 2 kilobases (Kb).

Comme le transposon utilisé code pour la résistance à la kanamycine, le fragment SacI de 2 Kb, contenant le transposon a pu être cloné dans le site SacI du plasmide pUC18 (VIEIRA J. et MESSING J., 1982. The PUC plasmids, an M13mp7- derived system for insertion mutagenesis and sequencing with synthetic universal primers. Gene, 19, 259-268). C'est en utilisant ce plasmide recombinant que les séquences nucléiques faisant l'objet de la présente d'invention ont été isolées.

L'ADN génomique de la souche Ty 2 a été digéré par l'endonucléase Sacl et les produits de cette restriction ont été ligaturés dans le site SacI du vecteur de clonage pUC18. Ce mélange de ligation a été utilisé pour transformer la souche Escherichia coli HB101 (déposée à la Collection de l'INSTITUT PASTEUR sous la référence CIP 102400). La sélection des transformants a été effectuée sur gélose nutritive contenant 100 µg/ml d'ampicilline. Après incubation durant 24 heures à l'étuve à 37°C, les transformants ont été repiqués sur des disques de nitrate de cellulose déposés sur des géloses nutritives contenant 100 µg/ml d'ampicilline. En vue de procéder à une hybridation in situ, ces disques ont ensuite été traités par une solution de dénaturation (NaOH 0,5M), puis par une solution de neutralisation (acétate d'ammonium IM, NaOH 0,02M). L'ADN libéré et dénaturé par ces traitements a été fixé sur les disques de nitrate de cellulose par chauffage à 80°C durant 3 heures.

Ces disques de nitrate de cellulose ainsi préparés ont été hybridés avec le fragment SacI de 2 kb contenant le transposon et marqué au ³²P. Les hybridations ont été réalisées à 65°C durant 24 heures en 6 x SSC (1 x SSC est constitué de 0,15 M Nacl et 0,015M citrate trisodique à pH 7) en milieu de Denhardt (0,1 % Ficoll ; 0,1 % polyvynil-pyrrolidone ; 0,1 % de sérum albumine de boeuf).

Il a été ainsi isolé un clone E. coli HB101 contenant un plasmide recombinant constitué du vecteur de clonage pUC18 dans lequel était inséré un fragment SacI de 2 kb (délimité par les sites S1 et S2 dans la carte de restriction de ce fragment qui est donnée dans la figure 1 en trait épais).

### 2. Région génétique associée au fragment BacI de 2 kb.

L'ADN génomique de la souche Ty 2 a été digéré par l'endonucléase HindIII. Les produits de digestion, séparés par électrophorèse en gel d'agarose, ont été transférés par capillarité sur feuille de nitrate de cellulose. Ils ont ensuite été hybridés dans les conditions décrites ci-dessus avec le fragment SacI-BamH1 de 1,1 kb (fragment A sur la figure 1) ou avec le fragment HindIII-EcoR1 de 1,3 kb (fragment B sur la figure 1) qui sont tous les deux internes au fragment SacI de 2 kb.

Il a été constaté que le fragment SacI de 2 kb était recouvert par deux fragments HindIII de taille respectivement égale à 2,3 kb et 5,6 kb sur le génome de la souche Typhi Ty 2 (soit un fragment de 7,9 kb délimité par les sites H₁ et H₂ sur la carte de restriction enzymatique de cette région qui est donnée dans la figure 1).

### 3. Isolement de la sonde pour Salmonella.

L'ADN du plasmide recombinant contenant le fragment SacI de 2 kb a été restreint par les endonucléases SacI et HindIII. Cette double digestion libère un fragment SacI-HIndIII de 487 paires de bases, qui a été recloné entre les sites de restriction SacI et HindIII du vecteur de clonage pUC18.

Ce fragment SacI-HindIII de 487 paires de bases est appelé ci-après "Sonde pour Salmonella" et correspond à la séquence nucléique (I) définie ci-dessus. La position de ce fragment SacI-HindIII dans le fragment Sacl de 2 kb est indiquée sur la figure 1.

### 4. Contrôle de la spécificité de la sonde pour Salmonella.

Pour effectuer ce contrôle, les souches utilisées ont été cultivées durant 24 heures à 37°C dans 200 µl de bouillon nutritif en plaques pour microtitration à 96 puits. A l'aide d'un inoculateur multi-points, ces souches sont remises en culture durant 5 heures à 37°C sur une feuille de nitrate de cellulose disposée sur une boite de gélose nutritive. La feuille de nitrate de cellulose est ensuite traitée et utilisée pour des expériences d'hybridation in situ.

Pour préparer la sonde pour Salmonella, le plasmide recombinant (pUC18 contenant le fragment SacI-HindIII de 487 paires de bases) a été digéré par les endonucléases SacI et HindIII. Après séparation des produits de cette digestion par électrophorèse en gel d'agarose, la sonde pour Salmonella a été purifiée par électro-élution. Elle a, ensuite, été marquée au ³²P par déplacement de césure.

Les expériences d'hybridation ont été réalisées à 65°C en 6 x SSC et en milieu de Denhardt pendant 18 heures de réaction. Dans ces conditions expérimentales, la spécificité de la sonde pour Salmonella a été contrôlée avec 768 souches bactériennes, qui se répartissent en 384 souches n'appartenant pas au genre Salmonella et 384 appartenant au genre Salmonella et représentant les sept sous-espèces du genre Salmonella . Ces 768 souches proviennent de la Collection du Centre Collaborateur de l'OMS de Référence et de Recherche pour les Salmonella et de la collection du Centre National pour les Salmonella .

Aucune des 384 souches n'appartenant pas au genre Salmonella n'a répondu avec la sonde pour Salmonella.

Sur les 384 souches de Salmonella, 382 ont répondu avec la sonde pour Salmonella. Les deux souches n'ayant pas répondu avec la sonde pour Salmonella appartiennent, l'une au sérotype Wedding et l'autre au sérotype Zuerich. Il s'agit dans les deux cas de la souche de référence du sérotype. Pour ces deux souches, il a été vérifié qu'elles sont non-invasives dans le modèle sur cellules HeLa en culture et qu'elles ne possédent ni le fragment homologue de la sonde pour Salmonella, ni même le fragment SacI de 2 kb.

### 5. Détection de Salmonella dans les produits biologiques après amplification génique.

Dans un produit biologique, on ne peut aujourd'hui détecter une bactérie appartenant à une espèce donnée parmi plusieurs dizaines de millions d'autres bactéries. Pour pouvoir mettre en évidence la présence d'une Salmonella dans un produit biologique polymicrobien à l'aide d'une sonde nucléotidique, il est indispensable de procéder :
- à une amplification du nombre de bactéries à détecter en mettant en culture l'échantillon à analyser ;
- et à une amplification du nombre de copies de la cible que l'on veut détecter avec la sonde nucléotidique en utilisant la technique de l'amplification génique (ou technique de la "Polymerase Chain Reaction" ou PCR).

Pour les Salmonella, le fragment SacI-HindIII de 487 paires de bases (la sonde pour Salmonella) est spécifique du genre Salmonella. Si, à partir de l'échantillon biologique à analyser, on met en évidence la présence de ce fragment, on pourra en déduire que l'échantillon étudié est contaminé par Salmonella.

### 5.1. Description des deux amorces utilisées pour la PCR.

A partir de la séquence nucléotidique (I) de la sonde pour Salmonella, deux amorces utilisées pour mettre en oeuvre la technique d'amplification génique ont été sélectionnées.

Les enchaînements de 17 bases correspondant aux deux amorces, désignées SS-1 et SS-2 dans la description ci-dessus, ont été utilisés pour amplifier le nombre de copies pour Salmonella.

5.2. Protocole utilisé avec les amorces se-1 et 88-2 pour la PCR.

Ce protocole é été mis au point en utilisant le kit "Gene amp" (marque déposée) de Perkin Elmer Cetus (réf. N 801-0055). Les amorces SS-1 et SS-2 ont été utilisées à la concentration finale de 200 pmole. Le mélange réactionnel a été réalisé selon les instructions du fabricant en utilisant 10 µl de la solution contenant l'ADN à amplifier sous un volume final de 50 µl. Ce mélange a été soumis à 20 cycles d'amplification en utilisant l'appareil "DNA thermal cycler" (marque déposée) de Perkin Elmer Cetus (réf. N801-0177) dans les conditions suivantes :
- étape de dénaturation à 94°C durant 120 secondes,
- étape de réassociation à 68°C durant 180 secondes,
- et étape d'élongation à 72°C durant 90 secondes.

L'ADN ainsi amplifié a été visualisé par électrophorèse (120 volts durant 30 minutes) en gel d'agarose contenant 2 % d'agarose à bas point de fusion (BRL ; réf. 5517) et % d'agarose normal (Sigma ; réf. A-6877). Après électrophorèse, l'ADN a été transféré sur feuille de nitrate de cellulose et hybridé avec la sonde pour Salmonella marquée au ³²P.

### 5.3. Spécificité et sensibilité de la technique

La souche LT2 de Salmonella sérotype Typhimurium, déposée à la Collection de l'Institut Pasteur sous la référence CIP 60.62T et la souche HB101 de E.coli (CIP 102.400) ont été utilisées pour étudier la spécificité et la sensibilité de la technique PCR dans les conditions expérimentales décrites ci-dessus.

La solution contenant l'ADN à amplifier a été préparée de la façon suivante :
1) les bactéries entières sont mises en suspension dans 100 µl d'eau distillée ;
2) la suspension bactérienne est traitée durant 10 minutes à 100°C dans un microtube pour centrifugation;
3) elle est ensuite centrifugée durant 3 minutes minutes à la vitesse maximale dans une microcentrifugeuse. En évitant le culot de centrifugation, on prélève 10 µl de cette solution pour procéder à l'amplification génique.

Lorsqu'on utilise une culture pure de la souche LT2 de Salmonella sérotype Typhimurium, on détecte l'ADN de 10 ou de moins de 10 bactéries dans 10 µl de solution soumise à l'amplification. Lorsqu'on utilise la souche HB101 de E. coli, aucune amplification n'est détectable même lorsqu'on travail avec l'ADN correspondant à 10⁶ bactéries dans 10 µl de solution soumise à l'amplification. Lorsqu'on réalise un mélange des deux souches bactériennes, on détecte l'ADN de 100 ou moins de 100 Salmonella mélangé avec l'ADN de 10⁶ E.coli HB101 dans 10 µl de la solution soumise à amplification.

### 5.4. Essai de détection de Salmonella dans un échantillon reconstitué au laboratoire.

L'échantillon utilisé est un lot de viande hachée destinée à l'alimentation animale. Il est très fortement contaminé par des bactéries à Gram positif ou négatif, aérobies ou anaérobies. Un comptage approximatif effectué sous microscope permet d'évaluer le nombre total de bactéries à 10⁹ par gramme de viande hachée. En numération sur boites de gélose nutritive, le nombre de bactéries aérobies cultivant sur ce milieu a été trouvé égal à 10⁸ par gramme de viande hachée. Cet échantillon ne contient pas de Salmonella : trois analyses effectuées avec les techniques classiques de bactériologie alimentaire se sont révélées négatives.

### 5.4.1. Préparation de l'échantillon pour analyse par PCR.

L'échantillon a été reconstitué en ajoutant un nombre variable de Salmonella sérotype Typhimurium (10, 10², 10³, 10⁶, 10⁵ ou 10⁶) à 25 g de viande hachée. Cet échantillon reconstitué est mis en culture dans 200 ml de bouillon trypto-caséine soja dans un erlen-meyer de 1 litre durant 18 heures à 37°C avec agitation. On traite de façon identique 25 g de viande hachée sans Salmonella.

Le lendemain, l'erlen-meyer est laissé durant 15 minutes sur la paillasse, sans agitation, à la température du laboratoire de façon à laisser sédimenter les plus gros débris. On prélève alors en surface 1 ml de bouillon que l'on transvase dans un microtube pour centrifugation et que l'on centrifuge durant 3 minutes à la vitesse maximale dans une microcentrifugeuse. Le surnageant est complètement éliminé à l'aide d'une pipette Pasteur. Le culot bactérien est remis parfaitement en suspension dans 100 µl d'eau distillée, puis passé durant 10 minutes à 100°C. On centrifuge de nouveau durant 3 minutes à la vitesse maximale. En évitant le culot de centrifugation, on prélève 10 µl de cette solution pour procéder à l'amplification génique dans les conditions déjà indiquées.

5.4.2. Spécificité et sensibilité de la technique.

Lorsqu'on part de 25 grammes d'échantillon sans Salmonella, on ne peut détecter aucune amplification de l'ADN contenu dans les 10 µl de la solution amplifiée. Lorsqu'on part de 25 grammes d'échantillon contenant 10² ou plus de 10² Salmonella, on détecte une amplification de l'ADN contenu dans les 10 µl de la solution amplifiée.

## Revendications

1. Acide nucléique isolé susceptible d'être utilisé comme sonde et/ou amorce pour la détection *in vitro* de bactéries du genre Salmonella, **caractérisé en ce qu'**il est susceptible de s'hybrider à 65°C en 6x SSC en milieu Denhardt, avec un fragment Hindlll de 7,9 kb de la souche *Salmonella Typhi* Ty2 comprenant l'enchaînement nucléotidique (I) suivant:

1
5′>CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3′
487

2. Acide nucléique isolé selon la revendication 1, **caractérisé en ce qu'**il est susceptible de s'hybrider à 65°C en 6x SSC en milieu Denhardt avec un fragment Sacl de 2 kb de la souche *Salmonella Typhi* Ty2 comprenant l'enchaînement nucléotidique (I).

3. Acide nucléique isolé selon la revendication 1, **caractérisé en ce qu'**il est susceptible de s'hybrider à 65°C en 6x SSC en milieu Denhardt, avec l'enchaînement nucléotidique (I) suivant:

1
5'>CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG

TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

4. Procédé de détection in vitro de la présence éventuelle de bactéries du genre Salmonella dans un échantillon biologique susceptible de les contenir **caractérisé en ce qu'**il comprend :
- le cas échéant, la mise en culture de l'échantillon,
- le cas échéant, l'amplification du nombre de copies de la séquence nucléique à détecter à l'aide d'un couple d'amorces nucléiques selon la revendication 1 à 3,
- la mise en contact de l'échantillon avec une sonde nucléique selon l'une des revendications 1 à 3, à 65°C en 6x SSC en milieu Denhardt,
- la détection éventuelle de complexes d'hybridation formés entre la sonde sus-mentionnée et la séquence nucléique à détecter.

5. Procédé selon la revendication 4, **caractérisé en ce que** l'étape d'amplification de la séquence nucléique à détecter, comprend les étapes suivantes :
- une étape d'extraction de l'acide nucléique à détecter appartenant au génome des bactéries du genre Salmonella éventuellement présentes dans l'échantillon biologique, et, le cas échéant, une étape de traitement à l'aide d'une transcriptase inverse dudit acide nucléique si ce dernier est sous forme d'ARN,
- un cycle comprenant les étapes suivantes :
• dénaturation de l'acide nucléique double brin à détecter, ce qui conduit à la formation d'un acide nucléique simple brin,
• hybridation de chacun des brins d'acide nucléique, obtenus lors de l'étape de dénaturation précédente, avec au moins une amorce selon l'une des revendications 1 à 3, par mise en contact des brins sus-mentionnés avec au moins un couple d'amorces sus-mentionnées,
• formation à partir des amorces des ADN complémentaires aux brins sur lesquels elles sont hybridées en présence d'une ADN polymérase et de quantités appropriées des quatre nucléosides triphosphate (dNTP) différents, ce qui conduit à la formation d'un plus grand nombre d'acides nucléiques double brin à détecter qu'à l'étape de dénaturation précédente, ce cycle étant répété un nombre de fois déterminé pour obtenir ladite séquence nucléique à détecter éventuellement présente dans l'échantillon biologique dans une proportion suffisante pour permettre sa détection.

6. Kit pour la détection de la présence éventuelle de bactéries du genre Salmonella dans un échantillon biologique **caractérisé en ce qu'**il comprend :
- le cas échéant, un milieu approprié pour la mise en culture de l'échantillon biologique,
- au moins un couple d'amorces nucléiques selon l'une des revendications 1 à 3,
- le cas échéant, des réactifs appropriés à la mise en oeuvre du cycle d'amplification, notamment de l'ADN polymérase, et des quantités appropriées des 4 nucléosides triphosphate différents,
- une (ou plusieurs) sonde(s) nucléique(s) selon l'une des revendications 1 à 3, pouvant être marquée, capable de s'hybrider avec la (ou les) séquence(s) nucléique(s) à détecter,
- le cas échéant des réactifs appropriés à la mise en oeuvre de la réaction d'hybridation entre la (ou les) sonde(s) et la (ou les) séquence(s) nucléique(s) à détecter susmentionnées.

7. Acide nucléique recombinant contenant au moins un acide nucléique isolé selon l'une des revendications 1 à 3, inséré dans un acide nucléique hétérologue vis-à-vis dudit acide nucléique isolé.

8. Acide nucléique recombinant selon la revendication 7 **caractérisé en ce que** l'acide nucléique isolé selon l'une des revendications 1 à 3 est précédé d'un promoteur (notamment un promoteur inductible) sous le contrôle duquel la transcription dudit acide nucléique isolé est susceptible d'être effectuée et, le cas échéant, suivi d'une séquence codant pour des signaux de terminaison de la transcription.

9. Vecteurs recombinants, notamment des plasmides, cosmides, ou phages, contenant un acide nucléique recombinant selon la revendication 7 ou la revendication 8, en l'un de leurs sites non essentiels pour leur réplication.

## Claims

1. An isolated nucleic acid which is capable of being used as a probe and/or primer for *in vitro* detection of bacteria of the genus *Salmonella,* **characterized in that** it is capable of hybridizing at 65°C, in 6x SSC, in Denhardt's medium, with a 7.9 kb HindIII fragment from the *Salmonella Typhi* Ty2 strain comprising the following nucleotide concatenation (I):

1
5' >CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATCCCTTAATATGTCGCCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

2. An isolated nucleic acid according to claim 1, **characterized in that** it is capable of hybridizing at 65°C, in 6x SSC, in Denhardt's medium with a 2 kb SacI fragment from the *Salmonella Typhi* Ty2 strain comprising the nucleotide concatenation (I).

3. An isolated nucleic acid according to claim 1, **characterized in that** it is capable of hybridizing at 65°C, in 6x SSC, in Denhardt's medium, with the following nucleotide concatenation (I):

1

5' >CAATTACGCCCCGATTATTATATCTCCGGCAGATGATACCCGAT

GGTAATGATAATATTGTACAGATCGAGATAGTTCGGGTTAAAGGT

TATCACCTGCTGCACCAGGAAAGCATTAAGTTGATAGAACACCAA

CCCGCTTCTCTCTTGCAAAACAAAATTGCGAATCTTTTGCTCAGA

TGTATTCCCGGACTTCGCTGGGACACAAAGCAAATTAGCGAGCTA

AATTCGATTGACAGTACCATGGTCTACTTACGCGGTAAGCATGAG

TTAAATCAATACACCCCCTATAGCTTACAGCAAGCGCTTAAATTC

CTGACTCAATCCCTTAATATGTCGCCCAAACAGCATTGCGCCTTAC

TGTGCGCTGGCAGAATGCTACCTCAGCATGGCGCAAATGGGGATT

TTTGATAAACAAAACGCAATGATCAAAGCTAAAGAACATGCGATT

AAGGCGACAGAGCTGGACCACAATAATCCACAAGCTT <3'

487

4. A method for *in vitro* detection of the possible presence of bacteria of the genus *Salmonella* in a biological sample which may contain them, **characterized in that** it comprises:
- as appropriate, culturing the sample;
- as appropriate, amplifying the number of copies of the nucleic acid sequence to be detected using a pair of nucleic acid primers in accordance with claims 1 to 3;
- bringing the sample into contact with a nucleic acid probe in accordance with one of claims 1 to 3, at 65°C, in 6x SSC, in Denhardt's medium;
- detecting, possibly, hybridization complexes formed between said probe and the nucleic acid sequence to be detected.

5. A method according to claim 4, **characterized in that** the step for amplification of the nucleic acid sequence to be detected comprises the following steps:
- a step for extracting the nucleic acid to be detected belonging to the genome of bacteria from the genus *Salmonella* which may be present in the biological sample and, if appropriate, a step for treating said nucleic acid using a reverse transcriptase, if the nucleic acid is in the form of RNA;
- a cycle comprising the following steps:
- denaturing the double strand nucleic acid to be detected, which results in the formation of a single strand nucleic acid;
- hybridizing each of the nucleic acid strands obtained during the preceding denaturing step with at least one primer in accordance with claims 1 to 3, by bringing said strands into contact with at least one of said pairs of primers;
- starting from primers, forming complementary DNA to the strands to which they are hybridized in the presence of a DNA polymerase and appropriate quantities of four different nucleoside triphosphates (dNTPs), which results in the formation of a larger number of double strand nucleic acids to be detected than in the preceding denaturing step;
this cycle being repeated a predetermined number of times to obtain said nucleic acid sequence to be detected which may be present in the biological sample in a proportion which is sufficient to allow its detection.

6. A kit for detecting the possible presence of bacteria of the genus *Salmonella* in a biological sample, **characterized in that** it comprises:
- if appropriate, a suitable medium for culturing the biological sample;
- at least one pair of nucleic acid primers according to any one of claims 1 to 3;
- if appropriate, reagents appropriate for carrying out the amplification cycle, in particular DNA polymerase, and appropriate quantities of 4 different nucleoside triphosphates;
- one (or more) nucleic acid probe(s) according to any one of claims 1 to 3, optionally labeled, capable of hybridizing with the nucleic acid sequence(s) to be detected;
- if appropriate, reagents appropriate for carrying out the hybridization reaction between said probe(s) and said nucleic acid sequence(s) to be detected.

7. A recombinant nucleic acid containing at least one isolated nucleic acid according to any one of claims 1 to 3, inserted in a nucleic acid which is heterologous as regards said isolated nucleic acid.

8. A recombinant nucleic acid according to claim 7, **characterized in that** the isolated nucleic acid according to any one of claims 1 to 3 is preceded by a promoter (in particular an inducible promoter) under the control of which transcription of said isolated nucleic acid is capable of being carried out and, if appropriate, followed by a sequence coding for transcription termination signals.

9. Recombinant vectors, in particular plasmids, cosmids or phages containing a recombinant nucleic acid according to claim 7 or claim 8, in one of their non essential sites for their replication.

## Patentansprüche

1. Isolierte Nukleinsäuresequenz, die geeignet ist, als Sonde und/oder Primer zum *in vitro*-Nachweis von Bakterien der Gattung Salmonella verwendet zu werden, **dadurch gekennzeichnet, dass** sie geeignet ist, in 6x SSC in Denhardt-Lösung bei 65°C mit einem HindIII-Fragment von 7,9 kb aus dem Stamm *Salmonella Typhi*Ty2 zu hybridisieren, welches die folgende Aneinanderreihung von Nukleotiden (I) aufweist:

1
5'>CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

2. Isolierte Nukleinsäure gemäß Anspruch 1, **dadurch gekennzeichnet, dass** sie in der Lage ist, in 6x SSC in Denhardt-Lösung bei 65°C mit einem SacI-Fragment von 2 kb aus dem Stamm *Salmonella Typhi* Ty2 zu hybridisieren, welches die Aneinanderreihung von Nukleotiden (I) umfasst.

3. Isolierte Nukleinsäure gemäß Anspruch 1, **dadurch gekennzeichnet, dass** sie in der Lage ist, in 6x SSC in Denhardt-Lösung bei 65°C mit folgender Aneinanderreihung von Nukleotiden (I) zu hybridisieren:

1
5'>CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTC
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT <3'
487

4. Verfahren zum *in vitro-*Nachweis der etwaigen Anwesenheit von Bakterien der Gattung *Salmonella* in einer biologischen Probe, welche geeignet ist, diese Bakterien zu enthalten, **dadurch gekennzeichnet, dass** es umfasst:
- gegebenenfalls die Probe in Kultur zu bringen,
- gegebenenfalls die Anzahl an Kopien der nachzuweisenden Nukleinsäuresequenz mit Hilfe eines Nukleinsäure-Primerpaares gemäß Anspruch 1 bis 3 zu amplifizieren,
- die Probe mit einer Nukleinsäure-Sonde gemäß einem der Ansprüche 1 bis 3 in 6x SSC in Denhardt-Lösung bei 65°C in Kontakt zu bringen,
- zwischen der oben erwähnten Sonde und der nachzuweisenden Nukleinsäuresequenz gebildete Hybridisierungskomplexe gegebenenfalls nachzuweisen.

5. Verfahren gemäß Anspruch 4, **dadurch gekennzeichnet, dass** der Schritt der Amplifizierung der nachzuweisenden Nukleinsäuresequenz die folgenden Schritte umfasst:
- einen Schritt einer Extraktion der nachzuweisenden Nukleinsäure, die aus dem Genom der Bakterien der Gattung *Salmonella* stammt, welche möglicherweise in der biologischen Probe vorhanden sind, und gegebenenfalls einen Schritt einer Behandlung der besagten Nukleinsäure mit Hilfe einer inversen Transkriptase, sofern die Nukleinsäure in Form von RNA vorliegt,
- einen Zyklus, der folgende Schritte umfasst:
• Denaturierung der nachzuweisenden doppelsträngigen Nukleinsäure, was zur Bildung einer einzelsträngigen Nukleinsäure führt,
• Hybridisierung jedes der bei dem vorhergehenden Schritt der Denaturierung erhaltenen Nukleinsäurestränge mit mindestens einem Primer gemäß einem der Ansprüche 1 bis 3, indem die oben erwähnten Stränge mit mindestens einem oben erwähnten Primerpaar in Kontakt gebracht werden,
• Bildung ausgehend von DNA-Primern, die komplementär zu den Strängen sind, mit denen sie hybridisiert sind, in Gegenwart einer DNA-Polymerase und geeigneter Mengen von vier verschiedenen Nukleosidtriphosphaten (dNTP), was zur Bildung einer größeren Anzahl an nachzuweisenden doppelsträngigen Nukleinsäuren als beim vorangegangen Schritt der Denaturierung führt,
wobei dieser Zyklus eine festgelegte Anzahl von Malen wiederholt wird, um die besagte nachzuweisende Nuleinsäuresequenz, die möglicherweise in der biologischen Probe vorhanden ist, in einem Ausmaß zu erhalten, das ausreicht, um deren Nachweis zu ermöglichen.

6. Kit zum Nachweis der etwaigen Anwesenheit von Bakterien der Gattung *Salmonella* in einer biologischen Probe, **dadurch gekennzeichnet, dass** es umfasst:
- gegebenenfalls ein geeignetes Medium, um die biologische Probe in Kultur zu bringen,
- mindestens ein Nukleinsäure-Primerpaar gemäß einem der Ansprüche 1 bis 3,
- gegebenenfalls geeignete Reagenzien zur Durchführung eines Amplifizierungszyklus, insbesondere DNA-Polymerase und geeignete Mengen der 4 verschiedenen Nukleosidtriphosphate,
- eine (oder mehrere) Nukleinsäuresonde(n) gemäß einem der Ansprüche 1 bis 3, die markiert sein können und die in der Lage sind, mit der (oder den) nachzuweisenden Nukleinsäuresequenz(en) zu hybridisieren,
- gegebenenfalls geeignete Reagenzien, um die Hybridisierungsreaktion zwischen der (oder den) Sonde(n) und der (oder den) oben erwähnten nachzuweisenden Nukleinsäuresequenz(en) durchzuführen.

7. Rekombinante Nukleinsäure, die mindestens eine isolierte Nukleinsäure gemäß einem der Ansprüche 1 bis 3 enthält, die in eine bezüglich der besagten isolierten Nukleinsäure heterologe Nukeinsäure insertiert ist.

8. Rekombinante Nukleinsäure gemäß Anspruch 7, **dadurch gekennzeichnet, dass** der isolierten Nukleinsäure gemäß einem der Ansprüche 1 bis 3 ein Promotor (insbesondere ein induzierbarer Promotor) vorangestellt ist, unter dessen Kontrolle die Transkription der besagten isolierten Nukleinsäure bewirkt werden kann, und der gegebenenfalls eine Sequenz folgt, die Transkriptionsterminationssignale codiert.

9. Rekombinante Vektoren, insbesondere Plasmide, Cosmide oder Phagen, die eine rekombinante Nukleinsäure gemäß Anspruch 7 oder Anspruch 8 in einer ihrer Stellen, die nicht für ihre Replikation essentiell sind, enthalten.
